# EUROPEAN PATENT APPLICATION

(11) **EP 4 272 698 A1**
(43) Date of publication of application: **08.11.2023**
(21) Application number: 22756470.5
(22) Date of filing: 15.02.2022
(51) Int. Cl.: A61F 2/06, A61L 27/50

(54) **ARTIFICIAL BLOOD VESSEL COMPLEX**

(30) Priority: 16.02.2021 KR 20210020772
(71) Applicant: The Asan Foundation, Seoul 05505 (KR); University Of Ulsan Foundation For Industry Cooperation, Ulsan 44610 (KR)
(72) Inventor: KIM, Joon Bum, Seoul 05507 (KR)
(74) Representative: BCKIP Part mbB
(86) International application number: PCT/KR2022/002239
(87) International publication number: WO 2022/177272

(57) **Abstract**

An artificial blood vessel complex includes: an artificial blood vessel tube member having a tube shape; and an artificial blood vessel ring member provided at an end of the artificial blood vessel tube member, for suturing with an aortic root through a suture, and having a curved shape with an inflection portion to correspond to a shape of an edge of the aortic root. The artificial blood vessel tube member includes: an artificial blood vessel tube portion; and a lounge tube portion extending from an end of the artificial blood vessel tube portion to be separated into a plurality of pieces, and having an end formed in a curved shape to match the artificial blood vessel ring member.

## Description

### Technical Field

The present disclosure relates to an artificial blood vessel complex.

### Background Art

In general, for the surgical treatment of aortic root dilation and consequent aortic valve insufficiency, Bentall operation using an artificial valve-artificial vessel complex is the most standardized treatment. However, the limited durability of valves due to implantation of prosthetic valves, the risk of bleeding due to use of anticoagulants, the risk of valve thrombosis-embolic complications and infective endocarditis have been pointed out as important problems.

To overcome the above deficiency, "valve-sparing root replacement" for replacing the aortic root while preserving the aortic valve has been proposed as an alternative, and research results showing clinical results that surpass the Bentall operation are accumulating.

However, the valve-sparing root replacement has technical difficulties and is difficult to generalize due to technical complexity. As a result, this operation is performed by only a few surgeons. In addition, in the case of valve-sparing root replacement, there are no consistent attachment points at which the residual aortic tissues will be attached to the inner walls of artificial blood vessels after the removal of the proximal aortic aneurysm, and thus structural distortion of the 3D structure of the valve and wrinkling of artificial blood vessels may occur during the process of suturing the residual tissues to the artificial blood vessel.

In addition, it is technically very difficult to fix the residual tissues to artificial blood vessels according to continuous suture in a narrow space inside the artificial blood vessels, and thus it takes a long time and bleeding is likely to occur. In particular, at the time of direct anastomosis of the residual tissue of the aortic root to flexible artificial blood vessels using a suture, it is technically difficult to suture along imaginary lines of the flexible artificial blood vessels in a narrow space, it is difficult to maintain consistency, and the risk of bleeding increases.

In addition, the diameter of the artificial blood vessel increases due to exposure to the systemic blood pressure for a long period of time, and accordingly, aortic valve function abnormality may occur due to change in the positional relationship of the aortic valve support structure, and there is a possibility of valve regurgitation.

### (Prior Art Document)

Korean Patent Laid-open Publication No. 10-2014-0010929 (published on 2020.05.19)

### Detailed Description of the Disclosure

### Technical Problem

Embodiments of the present disclosure are intended to provide an artificial blood vessel complex that can improve the accuracy of operation and the convenience of surgeons and reduce operation time and complications.

### Technical Solution to the Problem

In accordance with an aspect of the present disclosure, there is provided an artificial blood vessel complex including: an artificial blood vessel tube member having a tube shape; and an artificial blood vessel ring member provided at an end of the artificial blood vessel tube member, for suturing with an aortic root through a suture, and having a curved shape with an inflection portion to correspond to a shape of an edge of the aortic root.

Further, the artificial blood vessel tube member may include: an artificial blood vessel tube portion; and a lounge tube portion extending from an end of the artificial blood vessel tube portion to be separated into a plurality of pieces, and having an end formed in a curved shape to match the artificial blood vessel ring member.

Further, the artificial blood vessel ring member may include: a core part; an elastic part surrounding the core part to be sutured with a suture; and a skin part surrounding the elastic part.

Further, the elastic part may be convexly formed from the core part to be inserted into the artificial blood vessel tube member to be sutured with the suture above the core part.

Further, the elastic part may be convexly formed from the core part in a direction away from the artificial blood vessel tube member to be sutured with the suture under the core part.

Further, the elastic part may be convexly formed from the core part radially inwardly of the artificial blood vessel ring member to be in close contact with an outer wall of the aortic root.

Further, a height ratio of a diameter of the blood vessel ring member to a lower diameter of the lounge tube portion may be 0.8 to 1.0.

Further, a ratio of an upper diameter of the artificial blood vessel tube portion to a lower diameter of the lounge tube portion may be 0.8 to 1.0.

Further, the core part may be made of an alloy material having a predetermined strength or more, and the curved shape of the artificial blood vessel ring member may be maintained.

### Advantageous Effects of the Disclosure

The embodiments of the present disclosure provide a method of collectively suturing sutures to residual aorta tissues (aortic root) and coupling the suture sutured to the remaining aorta tissues to a pre-specified three-dimensional structure, for example, a blood vessel ring member of an artificial blood vessel complex, and thus have advantages in that it is technically easy to perform operation and structural consistency can be maintained.

Further, in the embodiments of the present disclosure, since it is important to maintain the height of a commissure to which the aortic valve is attached in maintaining the function of the aortic valve according to aortic valvuloplasty, the height is determined in three dimensions using a solid blood vessel ring member, and thus there is an advantage of aiding in maintaining the function of the aortic valve.

In addition, the embodiments of the present disclosure have advantages in that the accuracy of operation and the convenience of surgeons can be improved and the effect of reducing operation time and complications can be expected.

### Brief Description of the Drawing

FIG. 1 is a state diagram showing a state in which an artificial blood vessel complex according to a first embodiment of the present disclosure has been sutured to an aortic root.
FIG. 2 is a perspective view showing the artificial blood vessel complex according to the first embodiment of the present disclosure.
FIG. 3 is a cross-sectional perspective view taken by cutting along a line "III-III" in FIG. 2 according to the first embodiment of the present disclosure.
FIG. 4 is a cross-sectional perspective view showing an artificial blood vessel ring member and an artificial blood vessel tube member according to a modified example of the present disclosure.
FIG. 5 is a state diagram showing a state before suturing between an artificial blood vessel ring member and an aortic root according to another modified example of the present disclosure.
FIG. 6 is a state diagram illustrating a state after suturing between the artificial blood vessel ring member and the aortic root according to the other modified example of the present disclosure.
FIG. 7 is a perspective view showing an artificial blood vessel complex according to a second embodiment of the present disclosure.
FIG. 8 is a perspective view showing an artificial blood vessel complex according to a third embodiment of the present disclosure.

### Best Mode for Carrying out the Disclosure

Hereinafter, specific embodiments for implementing the idea of the present disclosure will be described in detail with reference to the accompanying drawings.

In describing the present disclosure, detailed descriptions of known configurations or functions may be omitted to clarify the present disclosure.

When an element is referred to as being 'connected' to, 'supported' by, 'accessed' to, 'supplied' to, 'transferred' to, or 'contacted' with another element, it should be understood that the element may be directly connected to, supported by, accessed to, supplied to, transferred to, or contacted with another element, but that other elements may exist in the middle.

The terms used in the present disclosure are only used for describing specific embodiments, and are not intended to limit the present disclosure. Singular expressions include plural expressions unless the context clearly indicates otherwise.

Further, in the present disclosure, it is to be noted that expressions, such as the upper side, the lower side, and the side surface are described based on the illustration of drawings, but may be modified if directions of corresponding objects are changed. For the same reasons, some components are exaggerated, omitted, or schematically illustrated in the accompanying drawings, and the size of each component does not fully reflect the actual size.

Terms including ordinal numbers, such as first and second, may be used for describing various elements, but the corresponding elements are not limited by these terms. These terms are only used for the purpose of distinguishing one element from another element.

In the present specification, it is to be understood that the terms such as "including" are intended to indicate the existence of the certain features, areas, integers, steps, actions, elements, combinations, and/or groups thereof disclosed in the specification, and are not intended to preclude the possibility that one or more other certain features, areas, integers, steps, actions, elements, combinations, and/or groups thereof may exist or may be added.

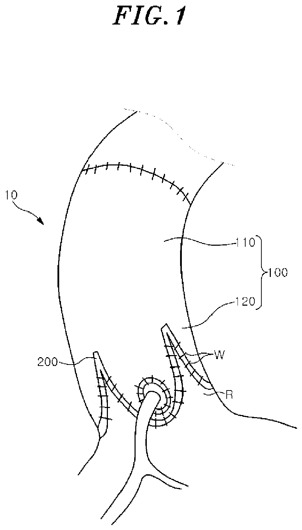

Hereinafter, a specific configuration of an artificial blood vessel complex according to embodiments of the present disclosure will be described with reference to FIGS. 1 to 8.

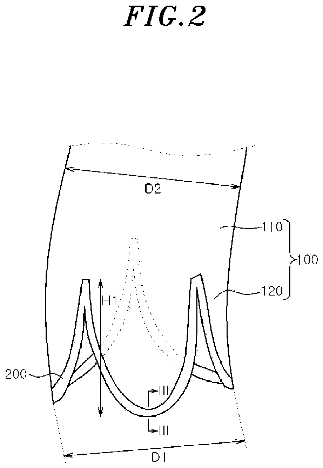

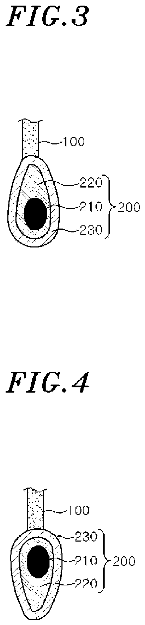

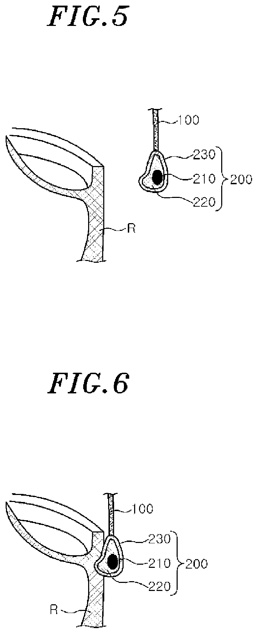

FIG. 1 is a state diagram showing a state in which an artificial blood vessel complex according to a first embodiment of the present disclosure has been sutured to an aortic root, FIG. 2 is a perspective view showing the artificial blood vessel complex according to the first embodiment of the present disclosure, FIG. 3 is a cross-sectional perspective view taken by cutting along a line "III-III" in FIG. 2 according to the first embodiment of the present disclosure, FIG. 4 is a cross-sectional perspective view showing an artificial blood vessel ring member and an artificial blood vessel tube member according to a modified example of the present disclosure, FIG. 5 is a state diagram showing a state before suturing between an artificial blood vessel ring member and an aortic root according to another modified example of the present disclosure, and FIG. 6 is a state diagram illustrating a state after suturing between the artificial blood vessel ring member and the aortic root according to the other modified example of the present disclosure.

As shown in FIGS. 1 to 6, the artificial blood vessel complex 10 according to the first embodiment of the present disclosure may be an artificially made blood vessel applicable to an aortic aneurysm. The artificial blood vessel complex 10 may be fixed to an aortic root R while maintaining a uniform three-dimensional positional relationship in the musculoskeletal structure of the aortic valve.

For example, the artificial blood vessel complex 10 can fix the height of the aortic root in a constant and stable manner as a whole by fixing the positional relationship between the sino-tubular junction and the nadir. In the present embodiment, the artificial blood vessel complex 10 fixes the positional relationship between the sino-tubular junction and the nadir, but it can also be applied to various positions in the body.

The artificial blood vessel complex 10 may include an artificial blood vessel tube member 100 provided in a tubular shape and an artificial blood vessel ring member 200 provided at an end of the artificial blood vessel tube member 100.

The artificial blood vessel tube member 100 may be in the form of a flexible artificial blood vessel. The artificial blood vessel tube member 100 may be made of a material capable of replacing biological blood vessels, for example, a polytetrafluoroethylene material, which is one of fluorine resins. In the present embodiment, the artificial blood vessel tube member 100 is a straight cylindrical tube member, but is not limited thereto, and the artificial blood vessel tube member 100 may have a form in which the distal end diameter of the vascular tube member 100 is convexly enlarged similarly to the actual aortic root.

The artificial blood vessel tube member 100 may include an artificial blood vessel tube portion 110 and a lounge tube portion 120. The artificial blood vessel tube portion 110 may be formed as a cylindrical hollow tube. The lounge tube portion 120 may be integrally formed at an end of the artificial blood vessel tube portion 110.

A plurality of (for example, three) lounge tube portions 120 extending from the end of the artificial blood vessel tube portion 110 may be provided. The lounge tube portion 120 may be formed in a curved shape having an inflection part as a whole. The artificial blood vessel ring member 200 may be connected to the lounge tube portion 120.

In particular, the upper end of the artificial blood vessel ring member 200 may continuously extend along the edge of lounge tube portion 120. Accordingly, the upper end of the artificial blood vessel ring member 200 may be provided in the form of a single straight line at a branch point where the lounge tube portion 120 branches into a plurality of pieces from the end of the artificial blood vessel tube portion 110. In this way, the upper end of the artificial blood vessel ring member 200 is formed in a continuous single straight line at the branch point of the lounge tube portion 120, and thus a commissure can be prevented from breaking when the artificial blood vessel complex 10 is attached to the aorta root R.

Further, the height of the straight portion of the artificial blood vessel ring member 200 at a portion where the upper end of the artificial blood vessel ring member 200 is formed in a single straight line at the branch point of the lounge tube portion 120 may be identical to or less than the height of the curved portion formed to be curved toward the lower side of the artificial blood vessel ring member 200.

The ratio D2/D1 of the upper diameter D2 of the artificial blood vessel tube portion 110 to the lower diameter D1 of the lounge tube portion 120 may satisfy a ratio of 0.8 to 1.0. If the ratio D2/D1 of the upper diameter D2 of the artificial blood vessel tube portion 110 to the lower diameter D1 of the lounge tube portion 120 is lower than 0.8, the flow path of the blood passing through the valve narrows while the blood flows through the upper end of the artificial blood vessel tube portion 110, and thus it may be difficult for the blood to smoothly flow in the artificial blood vessel tube portion 110.

The ratio H1/D1 of the height H1 to the lower diameter D1 of the lounge tube portion 120 may satisfy a ratio of 0.8 to 1.0. Here, the height H1 of the lounge tube portion 120 is the distance between the lowest position and the highest position of the lounge tube portion 120, and the height H1 of the lounge tube portion 120 may be understood as corresponding to the distance between the lowest and highest positions where the aortic valve joins the aorta with respect to the diameter of the aortic annulus.

It is desirable that the ratio of the height H1 to the lower diameter D1 of the lounge tube portion 120 be maintained at a constant ratio regardless of the pressure in the aortic lumen. In particular, when the ratio of the height H1 to the lower diameter D1 of the lounge tube portion 120 satisfies a ratio of 0.8 to 1.0, it may be most ideal in terms of valve joint and stress applied to the valve. When the ratio of the height H1 to the lower diameter D1 of the lounge tube portion 120 is lower than 0.8 or higher than 1.0, it may be difficult to prevent valve regurgitation.

The artificial blood vessel ring member 200 may be formed in a curved shape in an up and down direction such that it has an inflection portion at an end of the artificial blood vessel tube member 100. The artificial blood vessel ring member 200 may correspond to the edge shape of the lounge tube portion 120 of the artificial blood vessel tube member 100 and may correspond to the shape of the edge of the aortic root R. The artificial blood vessel ring member 200 may be sutured to the aortic root R through a suture W. The artificial blood vessel ring member 200 may include a core part 210, an elastic part 220, and a skin part 230.

The core part 210 may be made of an alloy material for supporting the structure of the aortic valve. For example, the core part 210 may have a solid three-dimensional ring shape made of an Elgiloy alloy (Co-Cr-Ni Alloy; non-magnetic cobalt-chromium-nickel-molybdenum alloy). As the core part 210 is made of an alloy material having a predetermined strength or more and has a solid three-dimensional ring shape, the curved shape of the artificial blood vessel ring member 200 can be strongly maintained and the positional relationship between the artificial blood vessel complex 10 and the lowest point of the valve can be stably maintained. Accordingly, it is possible to improve the durability of the aortic valve.

The elastic part 220 may have a structure that surrounds the core part 210 as a whole such that suturing using the suture W can be performed. The elastic part 220 may be made of an elastic material through which a suture needle (not shown) and the suture W can pass. For example, the elastic part 220 may be made of a silicone rubber material through which a suture needle and the suture thread W can pass. Since the elastic part 220 is formed to convexly protrude upward from the core part 210 so as to be inserted into the artificial blood vessel tube member 100, the suture W can be sutured to the elastic part 220 located on the upper side of the core part 210.

Although the elastic part 220 is formed to protrude upward from the core portion 210 in the present embodiment, the present disclosure is not limited thereto, and the elastic part 220 may be formed to protrude downward from the core part 210, as shown in FIG. 4, or may be formed to protrude convexly from the core part 210 in the lateral direction, as shown in FIGS. 5 and 6. In particular, when the elastic part 220 is formed to protrude convexly from the core part 210 in the lateral direction, the elastic part 220 elastically presses the outside of the aortic root R when the artificial blood vessel complex is fixed to the aortic root R, and thus it is possible to prevent blood from leaking into the space between the artificial blood vessel complex 10 and the aortic root R in advance.

In addition, as the elastic part 220 surrounds the solid three-dimensional ring-shaped core part 210 and the suture W is configured to pass through the elastic part 220, the curved shape of the artificial blood vessel ring member 200 can be firmly maintained with only a single suture line. Accordingly, in the conventional surgical method requiring a double suture, a high degree of skill was required at the time of the second suture, but the artificial blood vessel complex 10 according to the embodiment of the present disclosure can improve the above problems of the conventional surgical method.

The skin part 230 may be an outer skin structure of the artificial blood vessel ring member 200, which surrounds the elastic part 220. The skin part 230 may be integrally molded with the elastic part 220. The skin part 230 may be made of a material through which a suture needle and the suture W can pass. For example, the skin part 230 may be made of a polyester knit fabric material.

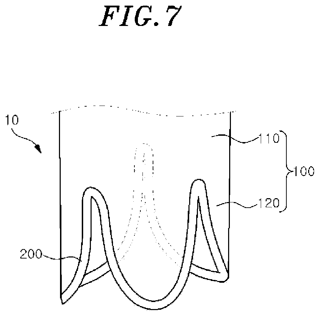

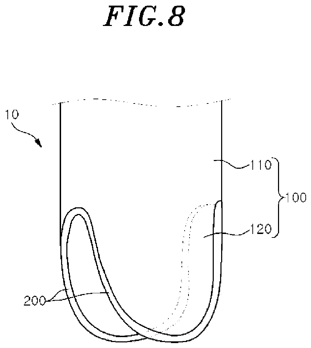

FIG. 7 is a perspective view showing an artificial blood vessel complex according to a second embodiment of the present disclosure and FIG. 8 is a perspective view showing an artificial blood vessel complex according to a third embodiment of the present disclosure.

As shown in FIG. 7, according to the second embodiment of the present disclosure, the artificial blood vessel complex 10 may include a curved artificial blood vessel ring member 200 formed to be curved in the up and down direction. Three inflection regions may be formed at the upper portion of the artificial blood vessel ring member 200, and three inflection regions may be formed at the lower portion of the artificial blood vessel ring member 200.

In addition, the lounge tube portion 120 of the artificial blood vessel tube member 100 may be formed in a curved shape corresponding to the artificial blood vessel ring member 200. Three lounge tube portions 120 branching off from the end of the artificial blood vessel tube portion 110 may be provided.

In addition, as shown in FIG. 8, according to the third embodiment of the present disclosure, the artificial blood vessel ring member 200 of the artificial blood vessel complex 10 may be provided in a shape curbed in the up and down direction. Two inflection regions may be formed at the upper portion of the artificial blood vessel ring member 200, and two inflection regions may be formed at the lower portion of the artificial blood vessel ring member 200.

The lounge tube portion 120 of the artificial blood vessel tube member 100 may be formed in a curved shape corresponding to the artificial blood vessel ring member 200. Two lounge tube portions 120 branching off from the end of the artificial blood vessel tube portion 110 may be provided.

Hereinafter, the operation and effects of the artificial blood vessel complex having the aforementioned configuration will be described.

When the artificial blood vessel complex 10 is applied to valve-sparing root replacement, the aortic tissue of the aortic root is removed first, and the coronary arteries are preserved with the openings thereof separated. Then, a suture W is connected to the residual aortic tissue adjacent to the aortic valve.

Thereafter, in a state where the artificial blood vessel complex 10 is placed at the position where the aortic tissue has been removed, the suture W connected to the residual aortic tissue (aortic root) is sutured to the artificial blood vessel ring member 200 of the artificial blood vessel complex 10 such that the suture W passes through. Then, the artificial blood vessel complex 10 is seated on the aortic annulus, and reconstruction is completed by tying a knot. In addition, the symmetry of the aortic valve is restored and the coronary arteries are re-connected.

As described above, according to the present disclosure, sutures are collectively sutured to the residual aortic tissue (aortic root), and the sutures sutured to the residual aortic tissue is coupled to a predetermined three-dimensional structure, for example, the blood vessel ring member of the artificial blood vessel complex, and thus it is technically easy and structural consistency can be maintained. Since it is important to maintain the height of a commissure to which the aortic valve is attached in maintaining the function of the aortic valve according to aortic valvuloplasty, the height is determined in three dimensions using the solid blood vessel ring member to aid in maintaining the function of the aortic valve. In addition, the accuracy of operation and the convenience of surgeons can be improved and the effect of reducing operation time and complications can be expected.

The embodiments described above are only a few examples of the technical idea of the present disclosure, and the technical scope of the present disclosure is not limited to the described embodiments. It will be possible for those skilled in the art to make various changes, modifications, and substitutions without departing from the technical scope of the present disclosure, and all such implementations should be appreciated as belonging to the technical scope of the present disclosure.

## Claims

1. An artificial blood vessel complex comprising:
an artificial blood vessel tube member having a tube shape; and
an artificial blood vessel ring member provided at an end of the artificial blood vessel tube member, for suturing with an aortic root through a suture, and having a curved shape with an inflection portion to correspond to a shape of an edge of the aortic root.

2. The artificial blood vessel complex of claim 1, wherein the artificial blood vessel tube member comprises:
an artificial blood vessel tube portion; and
a lounge tube portion extending from an end of the artificial blood vessel tube portion to be separated into a plurality of pieces, and having an end formed in a curved shape to match the artificial blood vessel ring member.

3. The artificial blood vessel complex of claim 1, wherein the artificial blood vessel ring member comprises:
a core part;
an elastic part surrounding the core part to be sutured with a suture; and
a skin part surrounding the elastic part.

4. The artificial blood vessel complex of claim 3, wherein the elastic part is convexly formed from the core part to be inserted into the artificial blood vessel tube member to be sutured with the suture above the core part.

5. The artificial blood vessel complex of claim 3, wherein the elastic part is convexly formed from the core part in a direction away from the artificial blood vessel tube member to be sutured with the suture under the core part.

6. The artificial blood vessel complex of claim 3, wherein the elastic part is convexly formed from the core part radially inwardly of the artificial blood vessel ring member to be in close contact with an outer wall of the aortic root.

7. The artificial blood vessel complex of claim 2, wherein a height ratio of a diameter of the blood vessel ring member to a lower diameter of the lounge tube portion is 0.8 to 1.0.

8. The artificial blood vessel complex of claim 2, wherein a ratio of an upper diameter of the artificial blood vessel tube portion to a lower diameter of the lounge tube portion is 0.8 to 1.0.

9. The artificial blood vessel complex of claim 3, wherein the core part is made of an alloy material having a predetermined strength or more, and the curved shape of the artificial blood vessel ring member is maintained.
